(19)

Europäisches
Patentamt
European
Patent Office
Office européen
des brevets

(11) **EP 3 051 277 A2**

(12) **EUROPEAN PATENT APPLICATION**

(43) Date of publication:
**03.08.2016 Bulletin 2016/31**

(51) Int Cl.:
**G01N 21/65** (2006.01) **B82Y 15/00** (2011.01)
**B82Y 20/00** (2011.01) **G01N 21/552** (2014.01)

(21) Application number: **16153339.3**

(22) Date of filing: **29.01.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **02.02.2015  JP 2015018433**

(71) Applicant: **Seiko Epson Corporation Tokyo 163 (JP)**

(72) Inventors:
• **Mano, Tetsuo**
  **Nagano 392-8502 (JP)**
• **Enari, Megumi**
  **Nagano 392-8502 (JP)**

(74) Representative: **Hoffmann Eitle Patent- und Rechtsanwälte PartmbB Arabellastraße 30 81925 München (DE)**

(54) **ELECTRIC-FIELD ENHANCEMENT ELEMENT, ANALYSIS DEVICE, AND ELETRONIC APPARATUS**

(57)    An electric-field enhancement element (100) includes a propagating surface plasmon generating unit (10); a localized surface plasmon generating unit (30) that is formed on an upper portion of a surface of a substrate and includes a plurality of fine metal structure bodies (32); and a metal layer (20) that is formed on an upper portion of the substrate surface and propagates a prop-agating surface plasmon generated by the propagating surface plasmon generating unit to the localized surface plasmon generating unit in a direction along the substrate surface, in which the fine metal structure body includes a plurality of fine metal structures (34) which are arranged to be separated from each other in a normal direction of the substrate surface.

FIG. 6

EP 3 051 277 A2

**Description**

BACKGROUND

1. Technical Field

The present invention relates to an electric-field enhancement element, an analysis device, and an electronic apparatus.

2. Related Art

In recent years, demands for medical diagnosis or testing for food have progressively increased, and the development of a sensing technique with a small size device and high speed is required. Various types of sensors including an electrochemical scheme are investigated, but interest in a sensor using surface plasmon resonance (SPR) has increased because of reasons such as capability of integration, low cost, and irrelativeness of a measurement environment.

For example, a scheme is known which detects whether or not a target substance is adsorbed, such as whether or not an antigen is adsorbed in an antigen-antibody reaction by using SPR generated on a metallic thin film that is provided on a surface of a total reflection prism. In this scheme, the presence of a detection target molecule is sensed by detecting the shift of extinction wavelengths due to the SPR before and after the adsorption of the detection target molecule (target substance).

Attention is paid to surface enhanced raman scattering (SERS) using SPR, as one of the high-sensitive spectroscopy techniques that detects the target substance having a low concentration. The SERS indicates a phenomenon in which raman scattered light is enhanced to be $10^2$ to $10^{14}$ times on a metal surface having a nanometer-scale unevenness structure.

When a molecule is irradiated with excitation light having a single wavelength such as a laser, light having a wavelength (raman scattered light), which is slightly shifted from the wavelength of the excitation light by the vibrational energy of the molecule is scattered. When a spectroscopic process is performed on the scattered light, a spectrum (fingerprint spectrum) unique to the molecular species is obtained. By analyzing the shape of the fingerprint spectrum, it is possible to identify the molecule.

Meanwhile, in the Raman spectroscopy, since the raman scattered light is extremely weak, various sensor chips, which show a strong enhancement factor, using the SERS are proposed for the detection of a substance having an extremely low concentration. For example, in JP-T-2007-538264 and JP-A-2008-014933, a chip is proposed which has a structure in which a metal nanoparticle layer is arranged on a mirror layer via a gap layer formed of a dielectric. Such a structure is also reported in OPTICS EXPRESS Vol.17, No.20 (2009) pp.17234-17241, and in the document, the structure is referred to as a gap type surface plasmon (GSP) model.

In the sensor chip described in the documents in the related art, the metal nanostructure formed on a substrate is irradiated with light to excite a localized surface plasmon (LSP) in the surrounding of the metal nanostructure, and thus the SERS is excited from the target molecule to perform high sensitive detection. However, in a case where further higher sensitive detection is attempted, it is important to enhance the electric field enhancement factor of a region in which an enhanced electric field is formed, that is, a so-called hot site (also referred to as hot spot) by more densely arranging the metal nanostructures, and to increase the number of the hot sites per unit area (density of hot site).

The hot site is generated in the surrounding of the metal nanostructure, and if the metal nanostructures are arranged to be adjacent to each other on the substrate, the hot site is generated with a higher enhancement factor between the adjacent metal nanostructures. In general, as the metal nanostructures approach each other, it is possible to improve the enhancement factor. In addition, in order to densely arrange the metal nanostructures on the substrate, a method of densely arranging the structures along the substrate surface and of arranging the structures to be stacked in a normal direction of the substrate surface is considered to be useful.

Further, in a case where the metal nanostructures are stacked in the normal direction of the substrate surface, the metal nanostructures in a vertical direction are separated by causing a dielectric to be interposed therebetween. In this case, the film thickness of the dielectric can be obtained by performing a film-forming process, and the film thickness of the dielectric can be formed to be thin with high accuracy. Therefore, it is possible to more easily arrange the metal nanostructures such that the distance therebetween is closer compared to a case in which the metal nanostructures are arranged on a plane along the substrate surface. Thus, by stacking the metal nanostructures in the normal direction of the substrate surface, it is expected to obtain a hot site showing a further strong electric field enhancement factor.

Meanwhile, in a case where excitation light having an electric field which vibrates in a direction along a line connecting the adjacent metal nanostructures is applied, in the hot spot, the electric field enhancement factor is further increased. That is, in order to obtain a high enhancement factor, it is useful for the excitation light to include polarized light in a direction along a line connecting the adjacent metal nanostructures.

Here, in a case where the metal nanostructures are arranged in a direction parallel to the substrate surface, by causing the excitation light to be incident on the substrate surface, it is easy to apply an electric field having an amplitude in a direction along an axis connecting pairs of the metal nanostructures. However, in a case of a structure in which the metal nanostructures are stacked in the normal direction of the substrate surface, it is favorable to use the excitation light having a polarized component in the normal direction of the substrate surface. If the excitation light is incident on the substrate surface at a right angle, such excitation light becomes light having a polarized component in a direction along the substrate surface in general, and thus, it is difficult to form a strong electric field between the pair of the metal nanostructures.

In order to apply an electric field having an amplitude component in a direction (that is, normal direction of the substrate surface) along an axis connecting the pair of metal nanostructures, the excitation light is necessarily incident on the substrate surface from a parallel direction, and in many cases, since the size of the pair of the metal nanostructures is significantly smaller compared to the spot diameter of light, only some of light is used for exciting the localized surface plasmon. Accordingly, the use efficiency of energy of the excitation light is decreased.

In this manner, in a case where the metal nanostructures are stacked in the normal direction of the substrate surface, it is difficult to sufficiently apply an electric field which vibrates in the normal direction of the substrate surface only by causing the excitation light to be incident on the substrate surface, and it is difficult to obtain the density of the hot site with an expected size.

## SUMMARY

An advantage of some aspects of the invention is to provide an electric-field enhancement element in which the density of a hot spot for adsorbing a target substance is high and an electric field enhancement factor of the hot spot is high, an analysis device, and an electronic apparatus.

The invention can be implemented as the following aspects or application examples.

An electric-field enhancement element according to an aspect of the invention includes a propagating surface plasmon generating unit; a metal layer that is formed on an upper portion of a surface of a substrate, and propagates a propagating surface plasmon generated by the propagating surface plasmon generating unit in a direction along the substrate surface; and a localized surface plasmon generating unit that is formed on an upper portion of the metal layer and at a position different from a position of the surface plasmon generating unit in a plan view, and includes a plurality of fine metal structure bodies, in which the fine metal structure body includes a plurality of fine metal structures which are arranged to be separated from each other in a normal direction of the substrate surface.

The electric-field enhancement element can apply, to the localized surface plasmon generating unit through the metal layer, an electric field having an amplitude in the normal direction of the substrate surface (light having a polarized component in the normal direction of the substrate surface) by propagating the propagating surface plasmon (PSP) generated by the propagating surface plasmon generating unit to the upper portion of the substrate surface. In this manner, it is possible to generate a strong localized surface plasmon (LSP) between the fine metal structures and it is possible to form hot sites having a high electric field enhancement factor in the vicinity of the fine metal structure. Therefore, in the electric-field enhancement element, a density of the hot spot for adsorbing a target substance is high, and the electric field enhancement factor of the hot spot is also high. Thus, it is possible to obtain a high enhancement factor of a SERS signal.

In addition, the propagating surface plasmon generating unit and the localized surface plasmon generating unit are formed at different positions in a plan view, in the electric-field enhancement element, the propagating surface plasmon is generated by incident light, and then the localized surface plasmon can be generated by the electric field of the propagating surface plasmon which has been propagated. If the excitation light is incident on the substrate surface at a right angle, the excitation light forms an optical electric field having a polarized component in a direction along the substrate surface and an amplitude of the electric field in the normal direction is less likely to be generated. However, it is possible to generate an enhanced electric field component in the normal direction of the substrate surface in a region to which the propagating surface plasmon is propagated, by propagating the propagating surface plasmon. In the electric-field enhancement element according to the aspect of the invention, the localized surface plasmon is generated in a pair of fine metal structures arranged in the normal direction of the substrate surface, by the electric field, and thus an extremely strong electric field can be formed between the pair of fine metal structures.

In the electric-field enhancement element according to the aspect of the invention, the fine metal structure bodies may be arranged in a grating shape in a direction along the substrate surface.

In the electric-field enhancement element with this configuration, the density of the hot site is high, and it is possible to generate the localized surface plasmon (hot site) having a high enhancement factor in the vicinity of the fine metal structure in a direction parallel to the substrate surface.

In the electric-field enhancement element according to the aspect of the invention, the metal layer may be continuous to the propagating surface plasmon generating unit.

According to the electric-field enhancement element with this configuration, it is possible to efficiently propagate the propagating surface plasmon generated by the propagating surface plasmon generating unit to the localized surface plasmon generating unit.

In the electric-field enhancement element according to the aspect of the invention, the propagating surface plasmon generating unit may generate the propagating surface plasmon by using a grating coupling method or an attenuated total reflection method.

According to the electric-field enhancement element with this configuration, it is possible to efficiently generate the propagating surface plasmon.

In the electric-field enhancement element according to the aspect of the invention, the propagating surface plasmon generating unit may include a grating and a surface on which the propagating surface plasmon is generated.

In the electric-field enhancement element with this configuration, it is possible to efficiently generate the propagating surface plasmon.

In the electric-field enhancement element according to the aspect of the invention, the propagating surface plasmon generating unit may include a prism and a surface on which the propagating surface plasmon is generated.

According to the electric-field enhancement element with this configuration, it is possible to efficiently generate the propagating surface plasmon.

An analysis device according to another aspect of the invention includes the electric-field enhancement element described above; a light source that irradiates the electric-field enhancement element with excitation light; and a detector that detects light emitted from the electric-field enhancement element.

According to the analysis device, since the analysis device includes the electric-field enhancement element having a high electric-field enhancing effect, the enhancement factor of light based on the plasmon is extremely great, and thus it is possible to analyze the target substance with extremely high sensitivity.

In the analysis device according to the aspect of the invention, the excitation light may be TM polarized light.

According to the analysis device, it is possible to efficiently generate the propagating surface plasmon by the propagating surface plasmon generating unit.

In the analysis device according to the aspect of the invention, a dimension of the fine metal structure in the normal direction of the substrate surface may be smaller than a wavelength of the excitation light.

An electronic apparatus according to still another aspect of the invention includes the analysis device described above; a computation unit that computes health care information based on detection information from the detector; a storage unit that stores the health care information; and a display unit that displays the health care information.

The electronic apparatus includes the electric-field enhancement element having a great enhancement factor of light based on the plasmon, and it is possible to easily detect the target substance and to provide health care information with high accuracy.

BRIEF DESCRIPTION OF THE DRAWINGS

The invention will be described with reference to the accompanying drawings, wherein like numbers reference like elements.

Fig. 1 is a schematic view illustrating an electric-field enhancement element according to an embodiment.

Fig. 2 is a schematic view illustrating an example of a propagating surface plasmon generating unit of an electric-field enhancement element according to an embodiment.

Fig. 3 is a schematic view illustrating an example of a propagating surface plasmon generating unit of an electric-field enhancement element according to an embodiment.

Fig. 4 is a schematic view illustrating an example of a propagating surface plasmon generating unit of an electric-field enhancement element according to an embodiment.

Figs. 5A to 5D are schematic views illustrating examples of a fine metal structure body of a localized surface plasmon generating unit of an electric-field enhancement element according to an embodiment.

Fig. 6 is a perspective view schematically illustrating an example of an electric-field enhancement element according to an embodiment.

Fig. 7 is a perspective view schematically illustrating an example of an electric-field enhancement element according to an embodiment.

Fig. 8 is a perspective view schematically illustrating an example of an electric-field enhancement element according to an embodiment.

Fig. 9 is a perspective view schematically illustrating an example of an electric-field enhancement element according to an embodiment.

Fig. 10 is a perspective view schematically illustrating an example of an electric-field enhancement element according to an embodiment.
Fig. 11 is a perspective view schematically illustrating an example of an electric-field enhancement element according to an embodiment.
Fig. 12 is a perspective view schematically illustrating an example of an electric-field enhancement element according to an embodiment.
Fig. 13 is a schematic view illustrating an exemplary cross section of an electric-field enhancement element according to an embodiment.
Figs. 14A and 14B are calculation examples of a hot site in a fine metal structure body.
Fig. 15 is a diagram schematically illustrating an analysis device according to an embodiment.
Fig. 16 is a diagram schematically illustrating an electronic apparatus according to an embodiment.

DESCRIPTION OF EXEMPLARY EMBODIMENTS

Hereinafter, several embodiments of the invention will be described. The embodiments described below are intended to describe an example of the invention. The invention is not limited by the embodiments, and includes various modification forms that are implemented in the scope in which the gist of the invention is not changed. All the configurations described below are not limited to be essential configurations of the invention.

1. Electric-field enhancement element

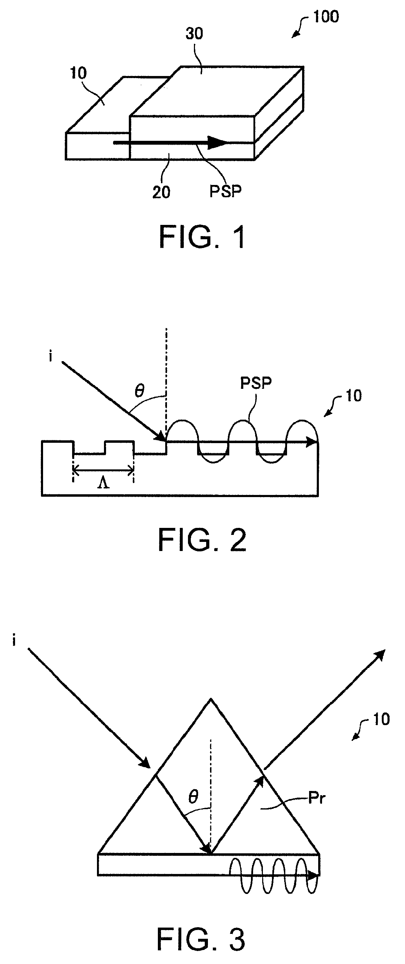

Fig. 1 is a perspective view schematically illustrating an electric-field enhancement element 100 of the embodiment which is an example of the electric-field enhancement element according to the invention. The electric-field enhancement element 100 of the present embodiment includes a propagating surface plasmon generating unit 10, a metal layer 20, and a localized surface plasmon generating unit 30. As illustrated in Fig. 1, the propagating surface plasmon (PSP) that is generated by the propagating surface plasmon generating unit 10 is propagated to the localized surface plasmon generating unit 30 through the metal layer 20 to cause the localized surface plasmon generating unit 30 to generate a localized surface plasmon (LSP).

In the present specification, when the term "upper portion" is used, for example, when "a specific object (hereinafter, referred to as 'B') is present on the 'upper portion' of another specific object (hereinafter, referred to as 'A')", the term "upper portion" is used to include a case in which B is present directly on A, and a case in which B is present on A through an object.

1.1. Propagating surface plasmon generating unit

The propagating surface plasmon generating unit 10 may be a substrate surface as is. The propagating surface plasmon generated by the propagating surface plasmon generating unit 10 may be generated to the upper portion of the substrate surface or may be generated to the lower portion of the substrate surface. The propagating surface plasmon generating unit 10 is configured of a metal in which an electric field, in which an electric field applied by light (for example, excitation light) and polarization induced by the electric field vibrate in opposite phases, is present, that is, a metal in which, when a specific electric field is applied, the real number part of a dielectric function has a negative value (has a negative dielectric constant), and the dielectric constant of the imaginary number part is smaller than the absolute value of the dielectric constant of the real number part. As the metal which has such a dielectric constant in a wavelength band of visible light, at least one type of metal selected from gold, silver, copper, aluminum, platinum, nickel, palladium, tungsten, rhodium, and ruthenium, or an alloy of plural types thereof and the like are exemplified.

The propagating surface plasmon generating unit 10 has a function of generating the propagating surface plasmon (PSP) in the electric-field enhancement element 100 of the present embodiment. When light is incident on the propagating surface plasmon generating unit 10, the propagating surface plasmon is generated in the vicinity of the surface (end face in the thickness direction) of the propagating surface plasmon generating unit 10.

As long as the propagating surface plasmon generating unit 10 has the surface described above, although not limited, a metallic thin film may be formed on the substrate to be used as the propagating surface plasmon generating unit 10, or the surface of the substrate of the metal may be used as the propagating surface plasmon generating unit 10. As the substrate in a case where a metallic thin film is formed on the substrate, a glass substrate, a silicon substrate, a resin substrate, an optical prism, and the like are exemplified. In this case, it is preferable that the substrate is less likely to be affected by the propagating surface plasmon excited by the propagating surface plasmon generating unit 10. The shape of the substrate surface is not particularly limited. In a case where a predetermined structure is formed on the surface of the propagating surface plasmon generating unit 10, the substrate may have a surface corresponding to the structure, or in a case where the surface of the propagating surface plasmon generating unit 10 is flat, the substrate may have a surface of which the corresponding part is flat.

Figs. 2 to 4 are schematic views respectively illustrating an example of the propagating surface plasmon generating unit 10. As long as the propagating surface plasmon generating unit 10 can generate the propagating surface plasmon (PSP), the propagating surface plasmon generating unit 10 may have any configuration and any shape.

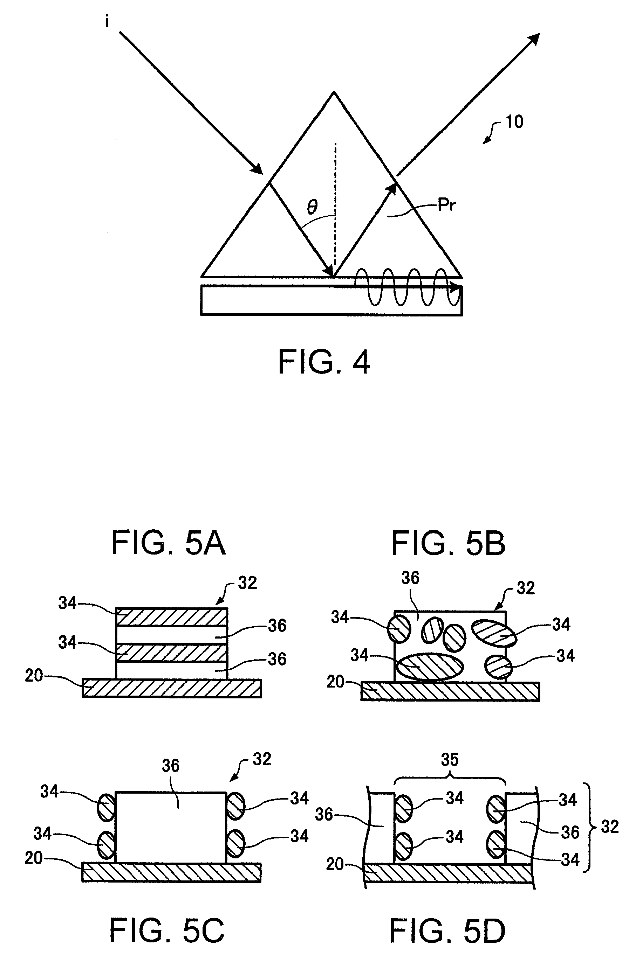

Fig. 2 schematically illustrates a state in which the PSP is generated by using a grating coupling method. Fig. 3 schematically illustrates a state in which the PSP is generated by the Kretschmann configuration among attenuated total reflection (ATR) methods. In this example, excitation light is incident on the metallic thin film through a prism Pr, and the PSP is generated in the vicinity of the surface of the metallic thin film on a side opposite to the prism Pr. In addition, Fig. 4 schematically illustrates a state in which the PSP is generated by the Otto configuration among the attenuated total reflection methods. In this example, excitation light is incident on the metallic thin film through the prism Pr, and the PSP is generated in the vicinity of the surface of the metallic thin film on a side of the prism Pr.

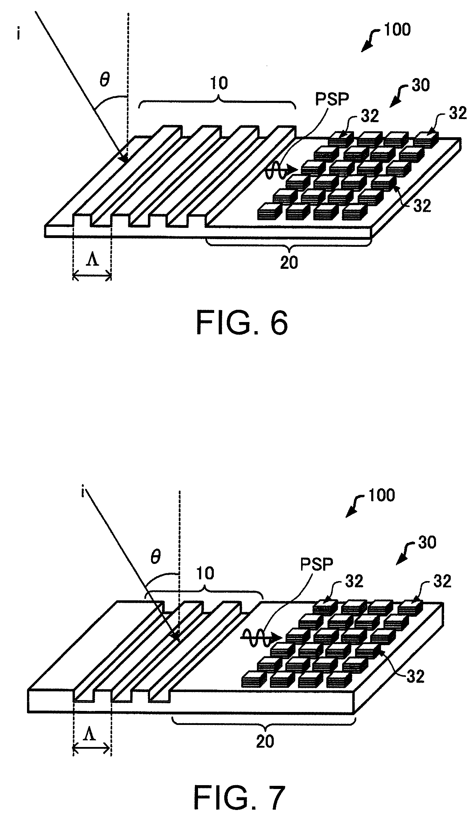

Figs. 6 to 12 are schematic views illustrating several examples of the electric-field enhancement element 100 that includes the propagating surface plasmon generating unit 10, the metal layer 20, and the localized surface plasmon generating unit 30. Figs. 6 to 12 each illustrate a case in which the propagating surface plasmon generating unit 10 is based on the grating coupling method.

The grating coupling method illustrated in Fig. 2 will be described. The pitch of the grating on the surface of the metal is set as A (refer to Figs. 2 and 6 to 12), and a wavevector $k_x$ of diffracted light, which is generated when excitation light i having a TM polarized component is incident, in a vertical direction with respect to the groove of the grating is expressed by Equation (1).

$$k_x = k_0 \sin\theta + mK \quad \ldots \quad (1)$$

Here, $k_0$ represents a wavenumber of the excitation light i, K represents a grating vector ($K = 2\pi/\Lambda$), $\theta$ represents an incident angle of the excitation light i (refer to Figs. 2 and 6 to 12), and m represents an integer.

In this system, when a relationship of $k_x > k_0$ is established, the diffracted light becomes evernescent waves, and when the wavenumber $k_x$ at this time and the wavenumber $k_{sp}$ of the surface plasmon are consistent with each other, the propagating surface plasmon is generated. The dispersion relationship of the propagating surface plasmon is obtained by the following equation.

$$k_{sp} = \frac{\omega}{c} \sqrt{\frac{\varepsilon_1 \varepsilon_2}{\varepsilon_1 + \varepsilon_2}} \quad \cdots (2)$$

Here, c represents a velocity of light in vacuum, $\varepsilon_1$ represents a dielectric constant of a medium on an incident side of the excitation light, and $\varepsilon_2$ represents a dielectric constant of the metal of the grating.

If the excitation light i includes the TM polarized component, the PSP can be generated. However, as the ratio of the TM polarized component in the excitation light i becomes great, energy of the excitation light i can be more efficiently converted into the PSP.

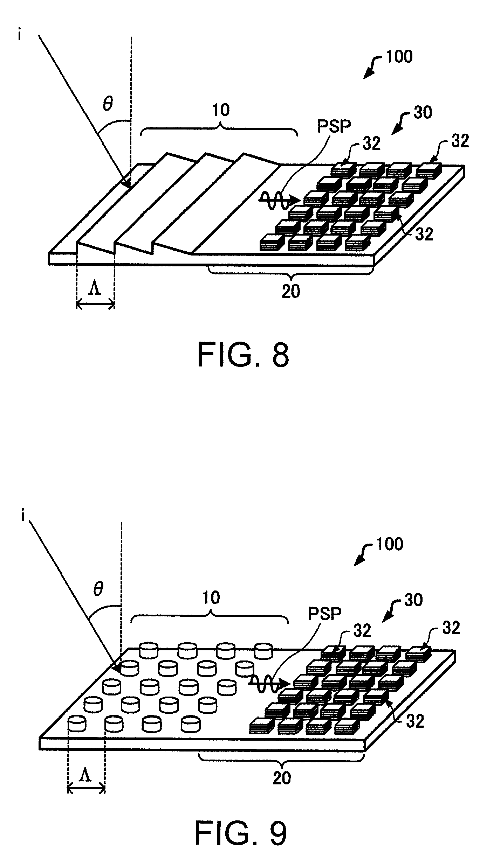

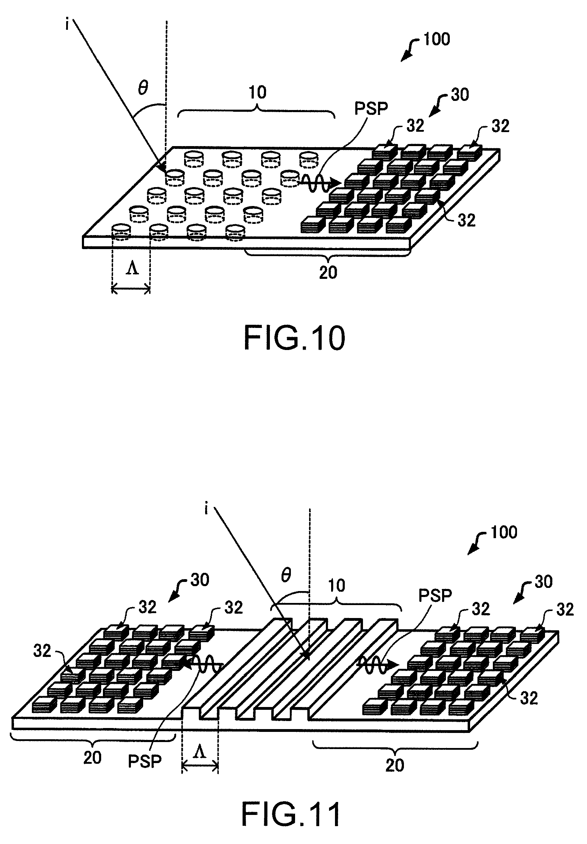

The shape of the grating is not particularly limited, and a structure in which convex parts are regularly arranged (refer to Figs. 6, 8, 9, 11, and 12), a structure in which concave parts are regularly arranged (refer to Figs. 7 and 10), and the like can be adopted. In addition, in Figs. 6 to 8 and 11, the propagating surface plasmon generating unit 10 has a one-dimensional grating structure in which linear convex parts or concave parts are regularly arranged, and in Figs. 9, 10, and 12, the propagating surface plasmon generating unit 10 has a two-dimensional grating structure in which punctiform (disk-shaped) convex parts or concave parts are regularly arranged.

Meanwhile, in the examples illustrated in Figs. 3 and 4, and Fig. 13 to be described below, the prism Pr is used. In cases of these examples, if Equation (3) is satisfied, the propagating surface plasmon is generated.

$$k_{sp} = \frac{\omega}{c} n_1 \sin\theta \quad \cdots (3)$$

Here, $n_1$ represents a refractive index of the prism Pr. In addition, the prism Pr and the surrounding of the metal layer 20 are set to be air (vacuum) having a refractive index of 1. The propagating surface plasmon (PSP) generated by the propagating surface plasmon generating unit 10 includes a component of an electric field which vibrates in a normal direction of the substrate surface. As illustrated in Fig. 1, the PSP is propagated to the localized surface plasmon generating unit 30 through the metal layer 20.

The structure or the shape of the propagating surface plasmon generating unit 10 is not limited to those exemplified above, and is arbitrary if the propagating surface plasmon generating unit 10 can generate the propagating surface plasmon (PSP).

1.2. Metal layer

The PSP generated by the propagating surface plasmon generating unit 10 is propagated to the localized surface plasmon generating unit 30 through the metal layer 20. The metal layer 20 is formed on the upper portion of the substrate surface (one surface of the substrate), and the PSP is propagated in a direction along the substrate surface in the vicinity of the surface of the metal layer 20. In this case, the substrate is a glass substrate, a silicon substrate, a resin substrate, or an optical prism, may be common with a substrate on which the propagating surface plasmon generating unit 10 is arranged.

In addition, the metal layer 20 may be a substrate surface as is. The metal layer 20 is configured of a metal. As the metal, at least one type of metal selected from gold, silver, copper, aluminum, platinum, nickel, palladium, tungsten, rhodium, and ruthenium, or an alloy of plural types thereof and the like are exemplified.

The metal layer 20 maybe adjacent to the propagating surface plasmon generating unit 10. In the examples illustrated in Figs. 6 to 12, the metal layer 20 is provided to be continuous to the metal of the propagating surface plasmon generating unit 10. In the examples illustrated in Figs. 6 to 12, the metal layer 20 is formed to be flat. In the present specification, the thickness direction of the metal layer 20 may be referred to as a depthwise direction or a height direction. In the present embodiment, in a case where the metal layer 20 is provided on the surface of the substrate, the normal direction of the surface of the substrate may be referred to as a depthwise direction or a height direction.

For example, the metal layer 20 can be formed by a method such as, deposition, sputtering, casting, and machining. The thickness of the metal layer 20 is not particularly limited as long as the propagating surface plasmon is propagated through the metal layer 20, and for example, the thickness can be set to be in a range of 10 nm to 1 mm, preferably 20 nm to 100 $\mu$m, and more preferably 30 nm to 1 $\mu$m. Further, the metal layer 20 is formed on the entire surface of the substrate, and a partial region thereof is formed with grating so that the region may be used as the propagating surface plasmon generating unit 10.

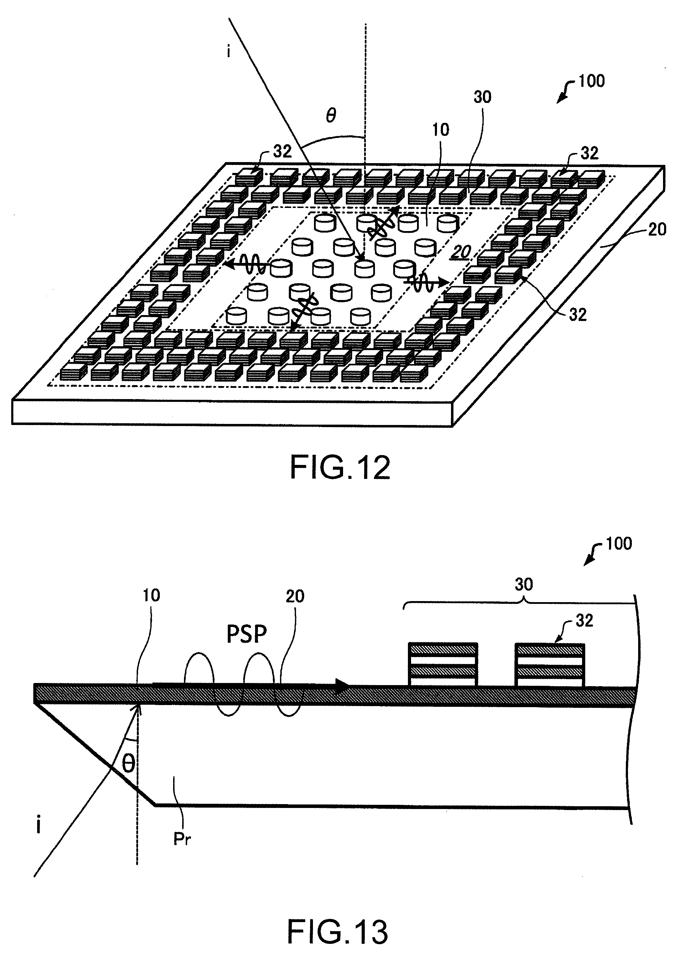

The planar arrangement of the propagating surface plasmon generating unit 10 and the metal layer 20 on the substrate surface is not particularly limited as long as the PSP can be propagated to the localized surface plasmon generating unit 30. Examples of such an arrangement include an arrangement in which the propagating surface plasmon generating unit 10 and the metal layer 20 are adjacent to each other in a plan view as illustrated in Figs. 6 to 10, an arrangement in which the propagating surface plasmon generating unit 10 is interposed between the metal layers 20 as illustrated in Fig. 11, an arrangement in which the propagating surface plasmon generating unit 10 is surrounded by the metal layer 20 as illustrated in Fig. 12, and the like.

In the examples illustrated in Figs. 6 to 12, since the propagating surface plasmon generating unit 10 adopts the grating coupling method, the generated propagating surface plasmon (PSP) is easily propagated in a predetermined direction along the substrate surface. Therefore, if the metal layer 20 and the localized surface plasmon generating unit 30 are arranged in a direction in which the PSP is propagated, the electric-field enhancement element 100 can be easily miniaturized and it is possible to increase the use efficiency of energy of excitation light.

For example, in the examples illustrated in Figs. 6 to 8, and 11, the metal layer 20 and the localized surface plasmon generating unit 30 are arranged in a direction in which the PSP is propagated (a direction perpendicular to the linear (one-dimensional) grating). In addition, in a case of the two-dimensional grating as the example illustrated in Fig. 12, it is preferable that the metal layer 20 and the localized surface plasmon generating unit 30 are arranged at least in directions perpendicular to an axis of each grating.

The PSP propagated through the metal layer 20 has an electric field component in the normal direction of the substrate surface (the thickness direction of the metal layer 20). Due to the electric field of the PSP, it is possible to form a region (the hot site) indicating a high electric field enhancement factor between a plurality of fine metal structures 34 of the localized surface plasmon generating unit 30.

Fig. 13 is a schematic view illustrating a cross section of another example of the electric-field enhancement element 100 that includes the propagating surface plasmon generating unit 10, the metal layer 20, and the localized surface plasmon generating unit 30. Fig. 13 illustrates a case where the propagating surface plasmon generating unit 10 adopts the Kretschmann configuration among the ATR methods.

Also in the example illustrated in Fig. 13, the PSP generated by the propagating surface plasmon generating unit 10 is propagated to the localized surface plasmon generating unit 30 through the metal layer 20. In the example illustrated in Fig. 13, the metal layer 20 is formed on one surface of the prism Pr (which can be regarded as the substrate), and the PSP is generated in the vicinity of the surface of the metal layer 20 on a side opposite to the prism Pr, and is propagated in a direction along the surface (the substrate surface) of the prism Pr. In this case, the substrate (the prism Pr) on which the propagating surface plasmon generating unit 10 is arranged is common with the prism Pr on which the metal layer 20 is arranged.

Also in the example illustrated in Fig. 13, the material, the thickness, and the planar arrangement of the metal layer 20 are the same as described in the examples illustrated in Figs. 6 to 12, and the metal layer 20 is adjacent to the propagating surface plasmon generating unit 10. In addition, the metal layer 20 as illustrated in Fig. 13 can be formed by performing the film formation of an appropriate metal on the prism Pr by deposition, sputtering, or the like.

Also in the example illustrated in Fig. 13, the PSP that is propagated through the metal layer 20 has an electric field component in the normal direction of the substrate surface (the thickness direction of the metal layer 20). Due to the electric field of the PSP, it is possible to form a region (the hot site) indicating a high electric field enhancement factor between the plurality of fine metal structures 34 of the localized surface plasmon generating unit 30. Although not illustrated, it is also the same as the case in which the propagating surface plasmon generating unit 10 adopts the Otto configuration among the ATR methods, in this case, the metal layer 20 is formed on one surface of the prism Pr (which can be regarded as the substrate), and the PSP is generated in the vicinity of an interface of the metal layer 20 and the prism Pr, and is propagated in a direction along the surface (the substrate surface) of the prism Pr.

1.3. Localized surface plasmon generating unit

The electric-field enhancement element 100 of the present embodiment includes the localized surface plasmon generating unit 30. The localized surface plasmon generating unit 30 is formed on the upper portion or the lower portion of the metal layer 20. That is, the localized surface plasmon generating unit 30 is formed on the surface side of the metal layer 20 to which the PSP generated by the propagating surface plasmon generating unit 10 is propagated. It is preferable that the localized surface plasmon generating unit 30 is formed on the surface or the interface side where the PSP of the metal layer 20 is present. In addition, the localized surface plasmon generating unit 30 is formed at a position different from that of the propagating surface plasmon generating unit 10 in a plan view. The localized surface plasmon generating unit 30 is configured to include a fine metal structure body 32.

Figs. 5A to 5D are schematic views illustrating several examples of the fine metal structure body 32. The fine metal structure body 32 includes the plurality of fine metal structures 34 which are arranged to be separated from each other in the normal direction of the substrate surface.

As illustrated in Figs. 5A to 5D, the fine metal structure 34 is arranged to be electrically separated from the metal layer 20. In the examples illustrated in Figs. 5A and 5B, the fine metal structure 34 is separated from the metal layer 20 by a dielectric 36, and further, the dielectric 36 is disposed between the plurality of fine metal structures 34 so that the fine metal structures 34 are arranged to be separated from each other. In the examples illustrated in Figs. 5C and 5D, the fine metal structures 34 are provided to the side surface of the dielectric 36 that is formed on the metal layer 20, and therefore the fine metal structures 34 are arranged to be separated from each other.

More specifically, in the example illustrated in Fig. 5A, the fine metal structure 34 and the dielectric 36 are arranged to be laminated in a layer shape, and the entirety thereof forms the fine metal structure body 32. In the example illustrated in Fig. 5B, the particulate fine metal structures 34 are dispersed on the dielectric 36, and the entirety thereof forms the fine metal structure body 32. In the example illustrated in Fig. 5C, the fine metal structure 34 is provided to the side surface of the columnar dielectric 36 provided on the metal layer 20, and the entirety thereof forms the fine metal structure body 32. In the example illustrated in Fig. 5D, the fine metal structure 34 is provided to the inner side surface of an opening 35 that is formed on the dielectric 36 provided on the metal layer 20, and the dielectric 36 and the fine metal structure 34 form the fine metal structure body 32. In Figs. 5A to 5D, although the examples are schematically illustrate, the number or the shape of the fine metal structure 34 included in the fine metal structure body 32 has no limitation. In addition, the fact that the fine metal structures 34 are arranged to be separated from each other in the normal direction of the substrate surface means that when the fine metal structure body 32 is projected in the normal direction, at least some of the separately arranged fine metal structures 34 overlap each other.

The dielectric 36 included in the fine metal structure body 32 has a function of arranging the fine metal structures 34 to be separately from each other in the fine metal structure body 32. However, the fine metal structures 34 included in the fine metal structure body 32 are not necessarily arranged to be separated from each other, and if some of the plurality of fine metal structures 34 are arranged to be separated from each other, the other fine metal structures 34 may be in contact with each other in the fine metal structure body 32. In a case where there are a plurality of fine metal structures 34 that are in contact with each other in the fine metal structure body 32, the plurality of fine metal structures 34 in contact with each other can be regarded as one fine metal structure 34, and a localized surface plasmon (LSP) is generated in the fine metal structure 34.

In addition, the dielectric 36 has a function of arranging the fine metal structures 34 to be separately from the metal layer 20. However, all the fine metal structures 34 may not be necessarily arranged to be separated from the metal layer 20, and some of the fine metal structures 34 may be in contact with the metal layer 20. By causing the fine metal structure 34 to be separated (insulated) from the metal layer 20, it is possible to generate the localized surface plasmon (LSP) in the fine metal structure 34.

The dielectric 36 has a function of structurally maintaining the plurality of fine metal structures 34. As long as the number of the fine metal structures 34 held by the dielectric 36 in one fine metal structure body 32 is two or more, there is no particular limitation. In addition, the fine metal structure 34 may be arranged to be contained in the fine metal structure body 32 by the dielectric 36, may be arranged to be exposed to the surface of the fine metal structure body 32, or may be arranged to be adsorbed to the surface of the fine metal structure body 32 (refer to Figs. 5B to 5D). In any structure, the dimension of the fine metal structure 34 is set to be smaller than the wavelength $\lambda$ of the excitation light i, and the localized surface plasmon resonance (LSPR) is generated so that the enhanced electric field is formed in the surrounding of the fine metal structure 34.

As the material of the dielectric 36, one type selected from silicon oxide ($SiO_2$), aluminum oxide ($Al_2O_3$), titanium oxide ($TiO_2$), tantalum pentoxide ($Ta_2O_5$), and silicon nitride (SiN) or a mixture of plural types thereof can be used. In a point that a vibrational electric field (light) due to the PSP easily reaches the fine metal structure 34 included in the dielectric 36, it is more preferable that the dielectric 36 is formed of a material through which light is transmitted.

The shape of the fine metal structure 34 is not particularly limited, and may be a plate shape, a disk shape, a spherical shape, a spheroid shape, or an indeterminate shape, or a shape obtained by combining those shapes. In addition, the plurality of fine metal structures 34 included in the fine metal structure body 32 may have different shapes.

It is preferable to set the size (dimension) of the fine metal structure 34 such that in a case where a sphere having the same volume as the fine metal structure 34 is assumed, the diameter of the sphere is smaller than the wavelength of the excitation light i. Specifically, it is preferable that the diameter of the sphere having the same volume as the fine metal structure 34 is equal to or greater than 5 nm and equal to or less than 450 nm. Further, it is preferable to set the dimension of the fine metal structure 34 such that the maximum span of the fine metal structure 34 is smaller than a half value of the wavelength (half wavelength) of the excitation light i.

The dimensions of the plurality of fine metal structures 34 included in the fine metal structure body 32 may be different from each other. Even in a case where the dimensions of the plurality of fine metal structures 34 are different from each other, the average dimension of the fine metal structures 34 can be defined. Accordingly, it is preferable to set the average size (average dimension) of the fine metal structures 34 such that in a case where a sphere having the same volume as the fine metal structure 34 is assumed, the average value (number average or volume average) of the diameters of the sphere is smaller than the wavelength of the excitation light i. Specifically, it is preferable that the average diameter of the sphere having the same volume as the fine metal structure 34 is equal to or greater than 5 nm and equal to or less than 450 nm. Further, it is preferable to set the dimension of the fine metal structure 34 such that the maximum span of the fine metal structure 34 is smaller than a half value of the wavelength (half wavelength) of the excitation light i.

In a case where the span of the fine metal structure 34 has a dimension approximately equal to or less than the half wavelength of the excitation light i, when the PSP reaches the fine metal structure 34, polarization of the electric field is generated in the fine metal structure 34, and thus the localized surface plasmon (LSP) is likely to be generated. In addition, the span of the fine metal structure 34 has a dimension approximately equal to the wavelength of the excitation light i, when the PSP reaches the fine metal structure 34, the polarization of the fine metal structure 34 becomes a state of a quadrupole, and thus it is possible to generate the LSP having an intensity lower than that in a case of the dimension approximately equal to or less than the half wavelength.

As the material of the fine metal structure 34, at least one type of metal selected from gold, silver, copper, aluminum, platinum, nickel, palladium, tungsten, rhodium, and ruthenium; an alloy of plural types thereof; or a complex of those metals or the alloy is exemplified. When such materials are used, a localized plasmon is likely to be generated due to light in the vicinity of the visible light. Among those materials, as the material of the fine metal structure body 32, gold or silver is preferable. If such materials are used, more strong LSP resonance is obtained and thus it is possible to improve the enhancement factor of the entirety of the element.

In a case where the fine metal structure body 32 adopts a configuration in which the fine metal structure 34 and the dielectric 36 are alternately stacked in a thin film shape as illustrated in Fig. 5A, the fine metal structure body 32 can be easily manufactured by using a photolithographic technique, a deposition technique, and the like which are general in the field of semiconductor manufacturing. Although not illustrated, for example, a metal is deposited on an appropriate substrate to form a metal film, and a grating structure is formed on a region to be the propagating surface plasmon generating unit 10 of the metal film, using a mask or the like by etching or the like. Then, the mask is appropriately formed on the region to be the localized surface plasmon generating unit 30 of the metal film, predetermined dielectrics and predetermined metals are alternately deposited, and then the mask is removed so that the fine metal structure body 32 can be manufactured. In this manner, it is possible to form the electric-field enhancement element 100 that includes the propagating surface plasmon generating unit 10, the metal layer 20, and the localized surface plasmon generating unit 30.

The fine metal structure body 32 as illustrated in Fig. 5B can be formed by a method for performing patterning such as a micro contact printing method and a nanoimprint method after the film formation by a multiple source deposition method, sputtering using a composite target, deposition, and the like. The fine metal structure body 32 can be formed by a lift-off method.

In addition, the fine metal structure body 32 as illustrated in Figs. 5C and 5D can be formed in a manner that a metal is deposited on an appropriate substrate to form a metal film, and a grating structure is formed on a region to be the propagating surface plasmon generating unit 10 of the metal film, using a mask or the like by etching or the like. Then, the dielectric is film-formed on the region to be the localized surface plasmon generating unit 30 of the metal film and a predetermined mask is formed so that a predetermined shape of the dielectric 36 is formed. Then, the fine metal structure body 32 can be manufactured by the deposition of a predetermined metal. In a deposition process of a metal, for example, the fine metal structure 34 having a predetermined size can be formed by adjusting a degree of vacuum, a substrate temperature, and the like.

The localized surface plasmon will be described. A case where a metal nanoparticle having a diameter D is assumed. In this case, if the diameter D is sufficiently lower than the wavelength $\lambda$ of the light i, when quasi-static approximation is taken into consideration, the relationship of Equation (4) is obtained for the polarizability $\alpha$ of polarization in particles exited by an optical electric field when light is incident on the metal nanoparticle.

$$\alpha \propto \frac{\varepsilon_1 - \varepsilon_2}{\varepsilon_1 + 2\varepsilon_2} \quad \cdots (4)$$

Here, $\varepsilon_1$ represents a dielectric constant of the surrounding of the metal nanoparticle, and $\varepsilon_2$ represents a dielectric constant of the metal. In a case where $\varepsilon_2$ has a negative value, $\alpha$ is drastically increased, specifically, in a case where $\varepsilon_2$ is - $(1/2)\varepsilon_1$, the denominator becomes 0, and thus the polarizability $\alpha$ becomes infinite in terms of a numerical expression. Practically, since the dielectric constant has an imaginary part, the polarizability has a finite value, but the value is extremely great. In accordance with the increasing of the polarizability, an extremely strong enhanced electric field is exited outside of the metal nanoparticle. Such a resonance phenomenon of polarization of light and metal nanoparticle is referred to as the localized surface plasmon resonance.

In the localized surface plasmon generating unit 30, the fine metal structure body 32 in which the fine metal structure 34 is arranged to be separated from the substrate surface in the normal direction (vertical direction) of the substrate surface, is formed on the upper portion of the metal layer 20 to which the propagating surface plasmon (PSP) is propagated. Then, since the PSP has an amplitude component of the electric field in the normal direction (vertical direction) of the substrate surface, hot sites having an extremely strong electric field enhancement factor are formed in the vicinity of the upper and lower end portions of the fine metal structure 34.

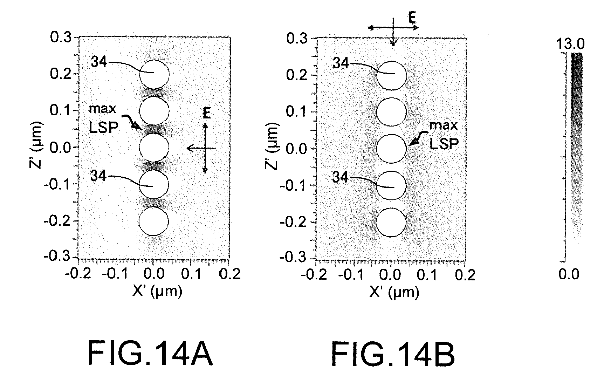

Figs. 14A and 14B illustrate calculation results indicating the distribution of the electric field enhancement factor in a model showing a state in which spherical fine metal structures 34 are arranged. Fig. 14A illustrates a case where an electric field (E in the drawing) which vibrates in a direction (Z' direction in the drawing) along the direction in which the fine metal structures 34 are arranged is applied, and Fig. 14B illustrates a case where an electric field (E in the drawing) which vibrates in a direction (X' direction in the drawing) perpendicular to the direction in which the fine metal structures 34 are arranged is applied.

In regard to Figs. 14A and 14B, in a case where an electric field which vibrates in a direction parallel to a direction in which the fine metal structures 34 approaches each other (direction along an axis connecting two fine metal structures 34) is applied, it can be known that a region to have an extremely great electric field enhancement factor is generated between the fine metal structures 34 (refer to an arrow in Fig. 14A). That is, in the electric-field enhancement element 100 of the present embodiment, the fine metal structures 34 are arranged to extremely approach each other in the normal direction of the substrate surface, and the LSP can be excited by the electric field, which vibrates in the normal direction of the substrate surface, applied by the PSP.

The planar arrangement of the localized surface plasmon generating unit 30 on the substrate surface is arbitrary in a range in which the PSP generated by the propagating surface plasmon generating unit 10 is propagated. Examples of such an arrangement include an arrangement in which the propagating surface plasmon generating unit 10 is adjacent to the metal layer in a plan view as illustrated in Figs. 6 to 10 and 13, an arrangement in which the propagating surface plasmon generating unit 10 is interposed as illustrated in Fig. 11, an arrangement in which the propagating surface plasmon generating unit 10 is surrounded as illustrated in Fig. 12, and the like.

1.4. Periodic arrangement of fine metal structure body

The fine metal structure body 32 may be periodically arranged. Here, the periodic arrangement means that the fine metal structure body 32 has a repetitive structure when the fine metal structure body 32 is planarly seen. The periodic arrangement can be evaluated by distinguishing the fine metal structure body 32 from portions (spaces) other than that, for example, in an image obtained by observing from the upper portion of the electric-field enhancement element 100.

In the examples illustrated in Figs. 6 to 12, the prism-shaped fine metal structure bodies 32 are periodically arranged in a grating shape in a plan view, a periodic structure is formed by such an arrangement. The periodic arrangement of the fine metal structure body 32 is not limited thereto.

In the examples illustrated in Figs. 6 to 12, when the fine metal structure body 32 is projected in the thickness direction of the metal layer 20 (in a plan view from the thickness direction), the shape of the fine metal structure body 32 can be a circle, an elliptical shape, a polygon, an indeterminate shape, or a shape obtained by combining those shapes. Even when the fine metal structure body 32 is projected in a direction perpendicular to the thickness direction, the shape of the fine metal structure body 32 can be a circle, an elliptical shape, a polygon, an indeterminate shape, or a shape obtained by combining those shapes. In the examples illustrated in Figs. 6 to 12, all of the fine metal structure bodies 32 are drawn to have a prism shape, but the shape of the fine metal structure body 32 is not limited thereto.

The size of the fine metal structure body 32 in the height direction refers to a length of a section in which the fine metal structure body 32 is cut by the plane perpendicular to the height direction, and is equal to or greater than 30 nm and equal to or less than 500 nm. In addition, in a case where the fine metal structure body 32 has a prism shape as illustrated in Figs. 6 to 12, it is preferable that the size of the fine metal structure body 32 in a direction perpendicular to the height direction is equal to or greater than 5 nm and equal to or less than 300 nm.

In the examples illustrated in Figs. 6 to 12, the fine metal structure bodies 32 are arranged in parallel in a grating shape. The number of the fine metal structure bodies 32 may be arbitrary as long as the periodic arrangement can be formed. Further, when the arrangement of the fine metal structure bodies 32 viewed from the thickness direction is regarded as a two dimensional lattice with the position of the fine metal structure body 32 as a lattice point, the irreducible fundamental unit lattice may be a rectangle or a parallelogram.

In addition, in the examples illustrated in Figs. 6 to 13, the fine metal structure body 32 is arranged to be in contact with the upper surface of the metal layer 20. However, a dielectric layer is formed on the upper surface of the metal layer 20, and the fine metal structure body 32 may be arranged thereon. In this manner, the metal layer 20 and the fine metal structure 34 can be arranged to be more accurately separated from each other. The material of such a dielectric layer may be the same as or different from that of the dielectric 36 of the fine metal structure body 32. In addition, the thickness when the dielectric layer is provided is arbitrary within a range in which the PSP present on the surface of the metal layer 20 can apply an electric field to the fine metal structure body 32.

1.5. Excitation light

In the electric-field enhancement element 100, at least the propagating surface plasmon generating unit 10 is irradiated with the excitation light i. The wavelength of the excitation light i incident on the electric-field enhancement element 100 is not limited as long as the excitation light i can cause the propagating surface plasmon generating unit 10 to generate the propagating surface plasmon. The excitation light i may be electromagnetic waves including ultraviolet radiation, visible light, and infrared radiation. In addition, the excitation light i may be or may be not polarized light, but if the excitation light i is TM polarized light, it is possible to efficiently generate the PSP.

The wavelength of the excitation light i is, for example, equal to or greater than 400 nm and equal to or less than 1,070 nm, preferably equal to or greater than 500 nm and equal to or less than 1,070 nm, and more preferably equal to or greater than 630 nm and equal to or less than 1, 070 nm. The size of a region to be irradiated with the excitation light i corresponds to a so-called spot size. The excitation light i may be converged by a lens or the like. In addition, the region to be irradiated with the excitation light i may have a circular shape or an elliptical shape in a plan view, or may have an arbitrary shape by using an aperture and the like. Further, when a lens or the like is used, aberration or the like may occur in the excitation light i.

1.6. Operational effects

The electric-field enhancement element 100 of the present embodiment can apply, to the localized surface plasmon generating unit 30 through the metal layer 20, an electric field having an amplitude in the normal direction of the substrate surface (light having a polarized component in the normal direction of the substrate surface) by the propagating surface plasmon (PSP) generated by the propagating surface plasmon generating unit 10. Accordingly, the strong localized surface plasmon (LSP) can be generated in plurality of fine metal structures 34 which are included in the fine metal structure body 32 of the localized surface plasmon generating unit 30, and are arranged to be separated in the normal direction of the substrate surface. Therefore, in the electric-field enhancement element 100, the density of the hot spot for adsorbing the target substance is high and the electric field enhancement factor of the hot spot can be increased. It is possible to obtain a strong enhancement factor of a SERS signal, and it is used for a high sensitive analysis with high accuracy.

Since the propagating surface plasmon generating unit 10 and the localized surface plasmon generating unit 30 are formed at different positions in a plan view, in the electric-field enhancement element 100 of the present embodiment, the propagating surface plasmon (PSP) is generated by the excitation light i, and then the localized surface plasmon (LSP) can be generated by the electric field of the propagating surface plasmon (PSP) which has been propagated. Here, if the excitation light i is incident on the substrate surface at a right angle, the excitation light i forms an optical electric field having a polarized component in a direction along the substrate surface and a vibrational electric field in the normal direction is unlikely to be generated. However, it is possible to generate an enhanced electric field component in the normal direction of the substrate surface in a region to which the PSP is propagated, by propagating the propagating surface plasmon (PSP). In the electric-field enhancement element 100 of the present embodiment, the localized surface plasmon (LSP) is generated in the fine metal structures 34 arranged in the normal direction of the substrate surface, by the electric field, and thus an extremely strong electric field can be formed between the fine metal structures 34 adjacent in the normal direction.

In addition, in the electric-field enhancement element 100 of the present embodiment, in a case where the fine metal structure bodies 32 are arranged in a grating shape in a direction along the substrate surface, it is possible to extremely increase the density of the hot site, and to generate the localized surface plasmon (hot site) having a high enhancement factor in the fine metal structure 34 in a direction parallel to the substrate surface.

2. Analysis device

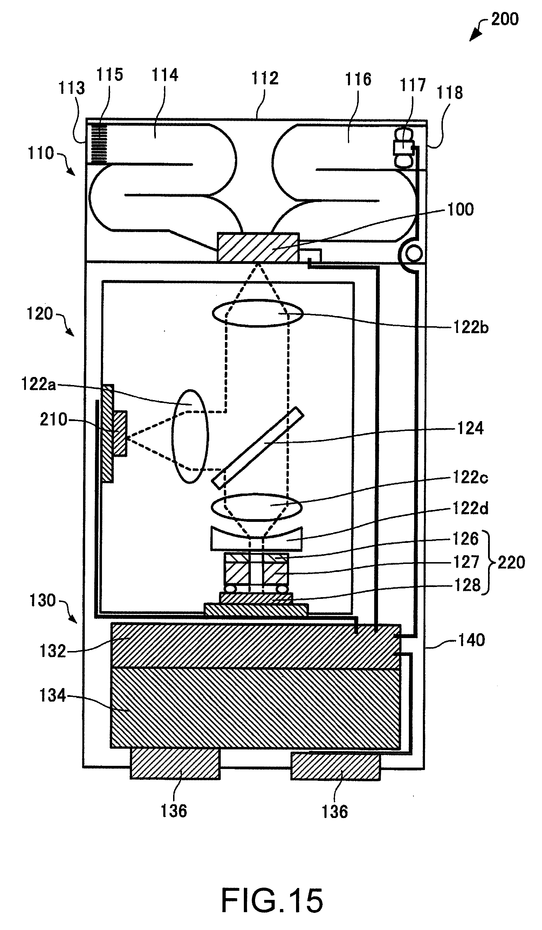

Fig. 15 is a diagram schematically illustrating an analysis device 200 according to the embodiment. For example, the analysis device 200 is a raman spectroscopic device, and the description will be made below by regarding the analysis device 200 as a raman spectroscopic device. As illustrated in Fig. 15, the analysis device 200 includes a gas specimen holding unit 110, a detecting unit 120, a control unit 130, and a casing 140 that accommodates the detecting unit 120 and the control unit 130. The gas specimen holding unit 110 includes the electric-field enhancement element according to the invention. Hereinafter, an example including the electric-field enhancement element 100 described above will be described.

The gas specimen holding unit 110 includes the electric-field enhancement element 100, a cover 112 that covers the electric-field enhancement element 100, a suction flow path 114, and a discharge flow path 116. The detecting unit 120 includes a light source 210, lenses 122a, 122b, 122c, and 122d, a half mirror 124, and a photoelectric detector 220. The control unit 130 includes a detection control section 132 that controls the photoelectric detector 220 by processing a signal detected by the photoelectric detector 220, and an electric power control section 134 that controls the electric power or the voltage of the light source 210 and the like. As illustrated in Fig. 15, the control unit 130 may be electrically connected to a connecting portion 136 that is for performing connection with the outside.

In the analysis device 200, when a suction mechanism 117 provided to the discharge flow path 116 is operated, the inside of the suction flow path 114 and the discharge flow path 116 becomes to have negative pressure, and a gas specimen including a target substance as a detection target through a suction port 113. A dust removing filter 115 is provided to the suction port 113, and can remove a powder dust having relatively large size, water vapor, or the like. The gas specimen passes through the suction flow path 114 and the discharge flow path 116, and is discharged through a discharge port 118. The gas specimen comes into contact with the fine metal structure body 32 of the electric-field enhancement element 100 when passing though those flow paths.

The suction flow path 114 and the discharge flow path 116 have a shape in which light is not incident from the outside on the electric-field enhancement element 100. In this manner, since light rays which are noise, other than the raman scattered light are not incident, it is possible to improve the S/N ratio of a signal. The material constituting the flow paths 114 and 116 is a material hardly reflects light or has a color hardly reflects lights.

The suction flow path 114 and the discharge flow path 116 have a shape in which the flow resistance with respect to the gas specimen becomes small. In this manner, high sensitive detection can be performed. For example, when the flow paths 114 and 116 have a smooth shape of which corners are eliminated as much as possible, it is possible to reduce the retention of the gas specimen at the corners. As the suction mechanism 117, for example, a pump or a fan motor of static pressure and an air flow depending on the flow resistance is used.

In the analysis device 200, the light source 210 irradiates the electric-field enhancement element 100 with light (for example, laser light or excitation light i having a wavelength of 633 nm). As the light source 210, for example, a semiconductor laser, or a gas laser can be used. The light emitted from the light source 210 is converged at the lens 122a, and then is incident on the electric-field enhancement element 100 through the half mirror 124 and the lens 122b. SERS light is emitted from the electric-field enhancement element 100, and the light reaches the photoelectric detector 220 through the lens 122b, the half mirror 124, and the lenses 122c and 122d. That is, the photoelectric detector 220 detects the light emitted from the electric-field enhancement element 100. Since the SERS light includes rayleigh scattered light having the same wavelength as the incident wavelength from the light source 210, the rayleigh scattered light may be removed by a filter 126 of the photoelectric detector 220. The light, from which the rayleigh scattered light is removed, is received as raman scattered light, by a light receiving element 128 through a spectroscope 127 of the photoelectric detector 220. As the light receiving element 128, for example, a photodiode is used.

For example, the spectroscope 127 of the photoelectric detector 220 is formed of an etalon or the like using Fabry-Perot resonance, and can cause the passing wavelength band to be variable. Raman spectrum unique to the target substance is obtained by the light receiving element 128 of the photoelectric detector 220, and for example, the signal intensity of the target substance can be detected by comparing the obtained Raman spectrum with data stored in advance.

The analysis device 200 is not limited to the above described example as long as the analysis device 200 includes the electric-field enhancement element 100, the light source 210, and the photoelectric detector 220, and causes the electric-field enhancement element 100 to adsorb the target substance to obtain the raman scattered light.

As described above, the analysis device 200 includes the electric-field enhancement element 100 in which the density per unit area of the hot spots is high and the electric field enhancement factor of the hot spot is high. Therefore, the intensity of raman scattered light can be increased. Accordingly, the analysis device 200 can have high detection sensitivity.

4. Electronic apparatus

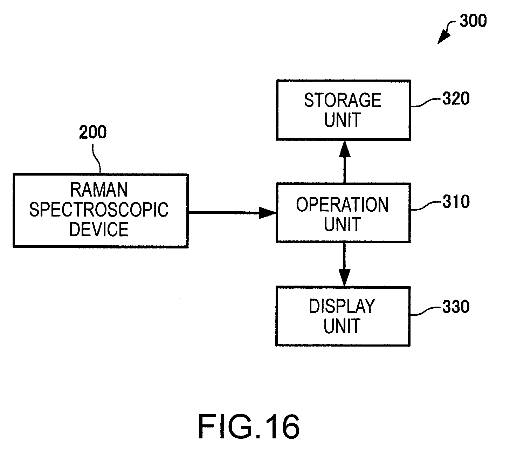

Next, an electronic apparatus 300 of the present embodiment will be described with reference to the drawings. Fig. 16 is a diagram schematically illustrating the electronic apparatus 300 according to the embodiment. The electronic apparatus 300 can include the raman spectroscopic device according to the invention. Hereinafter, an example in which the analysis device 200 is included as the raman spectroscopic device according to the invention will be described.

As illustrated in Fig. 16, the electronic apparatus 300 includes the analysis device 200, a computation unit 310 that computes health care information based on detection information from the photoelectric detector 220, a storage unit 320 that stores health care information, and a display unit 330 that displays health care information.

The computation unit 310 is, for example, a personal computer or a personal digital assistance (PDA), and receives detection information (signal or the like) transmitted from the photoelectric detector 220. The computation unit 310 computes health care information based on the detection information from the photoelectric detector 220. The computed health care information is stored in the storage unit 320.

The storage unit 320 is, for example, a semiconductor memory, a hard disk drive, or the like, and may be integrally configured with the computation unit 310. The health care information stored in the storage unit 320 is transmitted to the display unit 330.

The display unit 330 is configured by, for example, a display board (liquid crystal monitor or the like), a printer, a luminous body, a speaker, and the like. The display unit 330 performs a display or alarm activation based on the health care information computed by the computation unit 310 so that a user can recognize the contents.

As the health care information, information related to the presence or absence, or the amount of at least one type of bio-related materials selected from a group consisting of a bacterium, a virus, a protein, a nucleic acid, and antigen·antibody, or at least one type of compounds selected from an inorganic molecule and an organic molecule can be included.

The electronic apparatus 300 includes the analysis device 200 which can easily respond to the change of the plasmon resonance wavelength. Therefore, in the electronic apparatus 300, it is possible to easily detect a trace material, and to provide more accurate health care information.

The embodiments and the modification examples described above are merely an example, the invention is not limited thereto. For example, the embodiments and the modification examples can be appropriately combined.

For example, the electric-field enhancement element according to the invention can be used as an affinity sensor for detecting whether or not a substance is adsorbed such as whether or not an antigen is adsorbed in the antigen-antibody reaction. In the affinity sensor, white light is incident on the sensor, a wavelength spectrum is measured by the spectroscope, and the absorption of a detection substance to a sensor chip can be detected with high accuracy by detecting a shift amount of the surface plasmon resonance wavelength due to the adsorption.

The invention is not limited to the embodiment described above, and further can be variously modified. For example, the invention includes a configuration substantially the same as the configuration described in the embodiments (for example, configuration in which the function, the method, and the result are the same, or a configuration in which the purpose and the effect are the same). The invention includes a configuration in which non-essential parts of the configuration described in the embodiments are substituted. In addition, the invention includes a configuration that exhibits the same operational effects as the configuration described in the embodiments, or a configuration in which the same purpose can be achieved. Further, the invention includes a configuration in which known techniques are added to the configuration described in the embodiments.

**Claims**

1.  An electric-field enhancement element comprising:

    a propagating surface plasmon generating unit;
    a metal layer that is formed on an upper portion of a surface of a substrate, and propagates a propagating surface plasmon generated by the propagating surface plasmon generating unit in a direction along the substrate surface; and
    a localized surface plasmon generating unit that is formed on an upper portion of the metal layer and at a position different from a position of the propagating surface plasmon generating unit in a plan view, and includes a plurality of fine metal structure bodies,

    wherein the fine metal structure body includes a plurality of fine metal structures which are arranged to be separated from each other in a normal direction of the substrate surface.

2.  The electric-field enhancement element according to Claim 1,
    wherein the fine metal structure bodies are arranged in a grating shape in a direction along the substrate surface.

3.  The electric-field enhancement element according to Claim 1 or 2,
    wherein the metal layer is continuous to the propagating surface plasmon generating unit.

4.  The electric-field enhancement element according to any one of Claims 1 to 3,
    wherein the propagating surface plasmon generating unit generates the propagating surface plasmon by using a grating coupling method or an attenuated total reflection method.

5.  The electric-field enhancement element according to Claim 4,
    wherein the propagating surface plasmon generating unit includes a grating and a surface on which the propagating surface plasmon is generated.

6.  The electric-field enhancement element according to Claim 4 or 5,
    wherein the propagating surface plasmon generating unit includes a prism and a surface on which the propagating surface plasmon is generated.

7.  An analysis device comprising:

    the electric-field enhancement element according to any one of Claims 1 to 6;
    a light source that irradiates the electric-field enhancement element with excitation light; and
    a detector that detects light emitted from the electric-field enhancement element.

8.  The analysis device according to Claim 7,
    wherein the excitation light is TM polarized light.

9.  The analysis device according to Claim 7 or 8, wherein a dimension of the fine metal structure in the normal direction of the substrate surface is smaller than a wavelength of the excitation light.

10. An electronic apparatus comprising:

    the analysis device according to any one of Claims 7 to 9;
    a computation unit that computes health care information based on detection information from the detector;
    a storage unit that stores the health care information; and
    a display unit that displays the health care information.

FIG. 1

FIG. 2

FIG. 3

FIG. 4

FIG. 5A        FIG. 5B

FIG. 5C        FIG. 5D

FIG. 6

FIG. 7

FIG. 8

FIG. 9

FIG.10

FIG.11

FIG.12

FIG.13

FIG.14A          FIG.14B

FIG.15

300

200

STORAGE
UNIT — 320

RAMAN
SPECTROSCOPIC
DEVICE

OPERATION
UNIT — 310

DISPLAY
UNIT — 330

# FIG.16

**EP 3 051 277 A2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007538264 T

- JP 2008014933 A

**Non-patent literature cited in the description**

- *OPTICS EXPRESS,* 2009, vol. 17 (20), 17234-17241